# FASCICULE DE BREVET EUROPEEN

(11) **EP 2 550 458 B1**
(45) Date de publication et mention de la délivrance du brevet: **15.11.2017**
(21) Numéro de dépôt: 11707884.0
(22) Date de dépôt: 01.02.2011
(51) Int. Cl.: F04D 27/02, A61M 1/00, G05D 16/00, F04C 28/06

(54) **INSTALLATION DE DISTRIBUTION DE VIDE AVEC DETECTION ET PILOTAGE AUTOMATIQUE DES REMONTEES EN PRESSION**
VAKUUM-DISTRIBUTIONSANLAGE MIT ERKENNUNG UND AUTOMATISCHER KONTROLLE VON DRUCKANSTIEGEN
VACUUM DISTRIBUTION SYSTEM WITH DETECTION AND AUTOMATIC CONTROL OF PRESSURE INCREASES

(30) Priorité: 25.03.2010 FR 1052147
(43) Date de publication de la demande: 30.01.2013
(73) Titulaire: Air Liquide Santé France, 75007 Paris (FR); L'Air Liquide Société Anonyme pour l'Etude et l'Exploitation des Procédés Georges Claude, 75007 Paris (FR)
(72) Inventeur: PITTET, François, F-92800 Puteaux (FR); PIEILLER, Paul, F-92800 Puteaux (FR)
(74) Mandataire: Pittis, Olivier
(86) Numéro de dépôt international: PCT/FR2011/050198
(87) Numéro de publication internationale: WO 2011/117489

(56) Documents cités:
- EP-A1- 1 026 567
- EP-A1- 1 977 712
- EP-A1- 2 058 520

## Description

L'invention porte sur une installation de secours conçue pour garantir l'alimentation en fluides médicaux, en particulier en vide, des réseaux de vide équipant les services hospitaliers et analogues accueillant des patients hautement dépendants, en cas de dysfonctionnement de l'alimentation en vide desdits réseaux, ainsi que le procédé de pilotage d'une telle installation.

Dans les bâtiments hospitaliers ou analogues, on utilise des réseaux de canalisations pour distribuer les fluides médicaux, en particulier l'oxygène, l'air médical et le vide, au sein des différentes pièces ou des différents services, tel que salles de soin, salles de réanimation, chambres etc..., dans lesquels ils sont utilisés pour le traitement des patients.

Le vide ou dépression est utilisé au sein des bâtiments hospitaliers pour réaliser des aspirations notamment de fluides biologiques, tel le sang, l'urine ou autres.

En cas de défaillance de l'alimentation en fluides médicaux, c'est-à-dire en gaz médical ou en vide, d'une canalisation ou d'un réseau de canalisations, il est connu de pouvoir remplacer temporairement la ou les sources de gaz ou de vide alimentant normalement cette canalisation ou ce réseau de fluides par une ou des sources de gaz ou de vide de secours.

Pour ce faire, on utilise des armoires fixes ou des unités mobiles de secours qui viennent se raccorder au réseau à secourir de manière à alimenter ledit réseau en gaz et/ou en vide.

Ainsi, le document EP-A-1977712 enseigne une unité fonctionnelle hospitalière mobile pour la distribution momentanée de fluides médicaux, comprenant une armoire montée sur roues renfermant plusieurs bouteilles de gaz médical, notamment de l'oxygène et de l'air médical, et une pompe d'aspiration du vide médical permettant de maintenir le réseau de canalisation en dépression. Des prises de distribution de fluide médical permettent de connecter l'unité au réseau de distribution de fluides médicaux d'un bâtiment hospitalier. Des moyens de surveillance et de pilotage sont prévus pour avertir d'un dysfonctionnement éventuel de l'unité.

Une telle unité de secours permet de pallier toute défaillance de la distribution de fluides médicaux à partir du moment où cette défaillance a été détectée, par exemple si le personnel médical constate une augmentation de la pression au sein du réseau de vide, c'est-à-dire un niveau de pression excessif ou une baisse de la dépression régnant dans le réseau de vide.

Cependant, si une telle unité est efficace et remplit parfaitement son rôle, elle ne permet pas de détecter automatiquement les défaillances du réseau de distribution de vide agencé dans un bâtiment hospitalier, ni d'y apporter une réponse automatique et rapide.

Or, comme on peut aisément l'imaginer, pouvoir réagir rapidement en cas de rupture d'alimentation en vide est primordial dans certains services hospitaliers, notamment dans les salles d'urgence, de soins ou d'opération par exemple.

Le problème qui se pose est d'améliorer les unités ou installations de distribution de fluides médicaux, en particulier de vide, de manière à pouvoir détecter automatiquement une défaillance du réseau de distribution de vide au sein d'un hôpital ou analogue, et à pouvoir pallier automatiquement une telle défaillance et ce, préférentiellement en avertissant l'utilisateur, c'est-à-dire le personnel soignant, qu'une défaillance a eu lieu et qu'une procédure d'urgence a été mise en route.

La solution de l'invention est une installation de distribution de vide comprenant :
- une ligne principale de vide comprenant un orifice d'entrée et un orifice de sortie,
- au moins une pompe à vide,
- une ligne annexe de soutirage de vide raccordée fluidiquement, via une extrémité amont, à la ligne principale de vide et via une extrémité aval, à la pompe à vide,
- un capteur de pression comportant une prise de pression gazeuse raccordée fluidiquement à la ligne principale de vide, et
- un système de pilotage raccordé au capteur de pression et commandant ladite une pompe à vide en réponse à au moins une mesure de pression opérée par le capteur de pression au sein de la ligne principale de vide,
dans laquelle au moins une partie de la ligne principale de vide, la ligne annexe de soutirage de vide, la pompe à vide, le capteur de pression, et le système de pilotage sont compris dans une armoire,
dans laquelle le système de pilotage comporte au moins un automate programmable, et dans laquelle un boîtier, relié au système de pilotage, reçoit toutes les informations du ou des automates du système de pilotage,
l'installation étant caractérisée en ce que :
- le système de pilotage est conçu pour et apte à :
   i) démarrer la pompe à vide, lorsque la pression mesurée par le capteur de pression est supérieure à une valeur-seuil de pression préfixée, ladite valeur seuil de pression étant inférieure à 1 bar, et
   ii) arrêter la pompe à vide après une durée de fonctionnement préfixée,
- et un vaccuostat de secours permet, en cas de dysfonctionnement du capteur de pression, de détecter automatiquement toute remontée de pression à une valeur-seuil préfixée et de commander la pompe à vide pendant une durée de fonctionnement préfixée, indépendamment du système de pilotage.

Selon le cas, l'installation de invention peut comprendre l'une ou plusieurs des caractéristiques suivantes :
- un premier dispositif anti-retour de gaz est agencé sur la ligne principale de vide.
- un deuxième dispositif anti-retour de gaz est agencé sur la ligne annexe de soutirage de vide.
- le capteur de pression réalise des mesures en continu ou alors à une fréquence donnée, par exemple une ou plusieurs fois par minute ou par heure.
- le système de pilotage est conçu pour et apte à démarrer la pompe à vide lorsque la pression mesurée par le capteur est supérieure à une valeur seuil de pression préfixée, ladite valeur seuil de pression étant inférieure à 1 bar. Plus précisément, le démarrage de la pompe à vide a lieu dès lors que la pression excède cette valeur-seuil préfixée ou alors, selon le cas, uniquement lorsque la pression excède non seulement cette valeur-seuil de dépression préfixée mais aussi une valeur de pression critique préfixée comprise entre ladite valeur-seuil de dépression et la pression atmosphérique. Dans le deuxième cas, la valeur-seuil et la valeur critique constituent en quelque sorte des « paliers de pression ».
- la ligne annexe de soutirage de vide comprend au moins un dispositif de filtration de gaz, en particulier un filtre anti-bactérien.
- elle comporte en outre un récipient de récupération de liquide. La présence d'un tel récipient est importante car il permet de récolter et d'évacuer facilement les liquides pouvant être entraînés par l'aspiration de la pompe.
- le système de pilotage comporte plusieurs automates programmables.
- au moins un dispositif anti-retour comprend un clapet anti-retour.
- le système de pilotage est conçu pour et apte à arrêter la pompe à vide après une durée de fonctionnement préfixée comprise entre 1 minute et 30 minutes, de préférence comprise entre 2 et 15 minutes.
- elle comporte une alarme sonore ou visuelle se déclenchant en réponse à une mesure par le capteur d'une valeur de pression supérieure ou égale à une valeur de pression seuil au sein de la ligne principale de vide.
- les dispositifs anti-retour, le récipient de récupération de liquide et le filtre, en particulier de type anti-bactérien, sont également compris dans l'armoire, en particulier une armorie fixe, c'est-à-dire non mobile.
- une découpe dans l'une des portes de l'armoire permet à l'utilisateur d'avoir accès à différents afficheurs, touches de commande ou analogues situés sur le boîtier relié au système de pilotage.
- le boîtier reçoit toutes les informations du ou des automates du système de pilotage et affiche les alarmes destinées aux utilisateurs.

L'invention porte également sur une canalisation de vide, c'est-à-dire en dépression (pression interne inférieure à 1 bar) reliant une source de vide, en particulier une ou plusieurs pompes à vide, à au moins un site utilisateur, ladite source de vide étant raccordée fluidiquement à une entrée de ladite canalisation de manière à pouvoir mettre ladite canalisation en dépression, caractérisée en ce que ladite canalisation comprend une installation selon invention et en ce que ladite canalisation est raccordée au moins à une entrée et à au moins une sortie d'une ligne principale de vide de cette installation. Par ailleurs, l'invention concerne également un procédé de pilotage d'une installation selon invention agencée sur une canalisation de vide selon l'invention, caractérisé en ce qu'il comporte les étapes de :
a) déterminer au moyen d'au moins un capteur de pression, la dépression instantanée régnant dans la ligne principale de vide de l'installation de distribution de vide,
b) comparer au moyen du système de pilotage, la valeur de dépression instantanée déterminée à l'étape a) avec au moins une valeur-seuil de vide préfixée, ladite valeur-seuil de vide préfixée étant inférieure à la pression atmosphérique, par exemple une valeur seuil de -340 mbar, et
c) commander au moyen du système de pilotage, une mise en route et un fonctionnement de la pompe à vide de l'installation, lorsqu'à l'étape b), on détermine que la valeur de dépression instantanée mesurée est supérieure à la valeur-seuil de vide préfixée, et extraire du gaz, au moyen de ladite pompe à vide, d'au moins une partie de la ligne principale et de la canalisation reliée fluidiquement à ladite ligne principale, de manière à faire diminuer la pression régnant dans ladite ligne principale et ladite canalisation d'acheminement de vide,
d) stopper la pompe à vide après une durée donnée, caractérisé en ce qu'en cas de dysfonctionnement du capteur de pression, un vaccuostat de secours détecte automatiquement toute remontée de pression à une valeur-seuil préfixée et commande la pompe à vide pendant une durée de fonctionnement préfixée, indépendamment du système de pilotage.

Selon le cas, le procédé de invention peut comprendre l'une ou plusieurs des caractéristiques suivantes :
- lorsqu'à l'étape b), on détermine que la valeur de dépression instantanée mesurée est supérieure à la valeur-seuil de dépression préfixée, on déclenche au moins une alarme sonore ou visuelle, ou les deux.
- à l'étape d) la durée donnée est de préférence comprise entre 1 et 30 min, typiquement entre 2 et 15 minutes.
- lorsqu'à l'étape b), on détermine que la valeur de dépression instantanée mesurée est supérieure à la valeur-seuil de dépression préfixée, la mise en route et le fonctionnement de la pompe à vide (4) de l'installation est commandée seulement lorsque le capteur (10) détermine une valeur de dépression instantanée est supérieure ou égale à une valeur critique préfixée, ladite valeur critique préfixée étant comprise entre ladite valeur-seuil de dépression et la pression atmosphérique.
- on répète cycliquement les étapes c) et d) tant que la valeur de dépression mesurée par le capteur est supérieure à la valeur-seuil de vide préfixée.

La présente invention va maintenant être décrite plus en détail en références aux Figures annexées parmi lesquelles :
- la Figure 1 représente une vue schématique d'une installation de distribution de fluides médicaux selon l'invention reliée au réseau de vide d'un bâtiment hospitalier,
- la Figure 2 représente une armoire d'une installation selon l'invention,
- la Figure 3 schématise un boîtier d'alarme de type VIGI 3077 susceptible d'équiper une armoire selon la Figure 2,
- la Figure 4 représente l'interface d'un automate utilisable dans le système de pilotage d'une installation selon l'invention, et
- la Figure 5 représente le principe de fonctionnement de l'installation de la Figure 1.

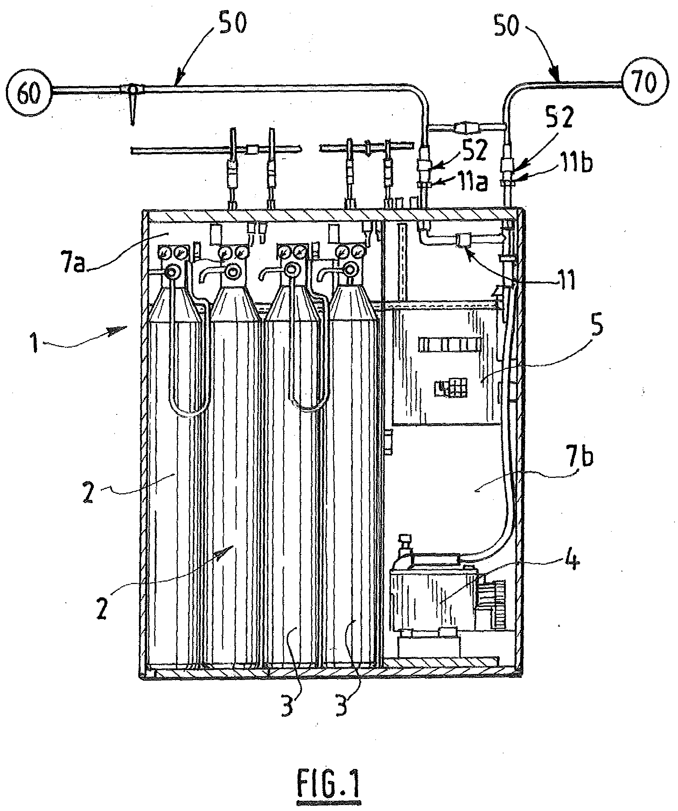

La Figure 1 représente une vue schématique d'une installation de distribution de fluides médicaux selon l'invention apte à et conçue pour détecter automatiquement une augmentation intempestive de la pression engendrant un niveau de vide inadéquat et pour réagit automatiquement à une telle détection.

Cette installation comprend une armoire 1 fixe reliée au réseau de canalisations 50 de vide au sein d'un bâtiment hospitalier entre une source de vide 60, typiquement une ou plusieurs pompes à vide agencées en parallèles tel que connu du document EP-A-1026567, et les sites utilisateurs 70 de vide, en particulier une ou plusieurs salles d'opération, salles de soins, salles de réanimation, chambres etc... Le vide est généralement utilisé au sein des bâtiments hospitaliers pour réaliser des aspirations notamment de fluides biologiques, tel le sang, l'urine ou autres.

De manière générale, dans le cadre de l'invention :
- par « vide », on entend une pression inférieure à la pression atmosphérique (< 1 bar ou < 1 atm), c'est-à-dire une dépression.
- par « mise sous vide », on entend diminuer la pression en dessous de la pression atmosphérique, c'est-à-dire à une pression inférieure à 1 bar.
- par canalisation ou réseau de vide, on entend une ou plusieurs canalisations véhiculant du vide, c'est-à-dire au sein de laquelle ou desquelles règnent une dépression, c'est-à-dire une pression inférieure à la pression atmosphérique (< 1 bar).
- on utilise indifféremment, les termes « ligne », « canalisation », « réseau » pour désigner une ou des conduites d'acheminement du vide.

Comme on le voit sur la Figure 1, l'armoire 1 comporte, dans sa partie gauche 7a, des bouteilles de gaz type B50 contenant des gaz médicaux à une pression pouvant aller jusqu'à 300 bar, à savoir deux bouteilles 2 contenant de l'oxygène et deux bouteilles 3 contenant de l'air médical, destinées à alimenter les réseaux de gaz médicaux de l'hôpital et, dans sa partie droite 7b, une pompe à vide 4 reliée au réseau de vide du bâtiment hospitalier, ainsi qu'un système de pilotage 5 apte à et conçu pour piloter la distribution des gaz provenant des bouteilles 2, 3 ou du vide produit par la pompe à vide 4, comme expliqué ci-après.

A titre d'exemple, on peut utiliser une pompe 4 à vide de 40 m³/h reliée au réseau de vide 50 du bâtiment.

Les bouteilles 2, 3 sont équipées de manière classique de robinets équipés de détendeurs en sortie et de manomètres indicateurs de pression permettant de réguler la pression du gaz en sortie de bouteilles 2, 3. Par exemple, on peut utiliser des bouteilles de type B50 contenant environ 10 m³ de gaz de manière à obtenir un débit maximal de l'ordre de 30 m³/h. Toutefois, les contrôles de l'alimentation en oxygène et en air de secours se faisant d'une manière différente de celui de la pompe à vide 4, ils ne seront pas détaillés ci-après et n'entrent pas dans le cadre de la présente invention.

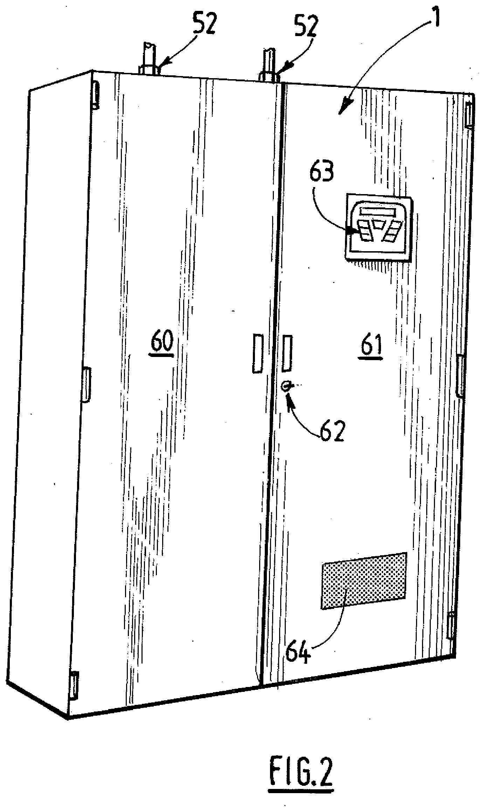

Comme visible sur la Figure 2, l'armoire 1 est munie de portes 60, 61 munies d'un système de verrouillage 62 classique, c'est-à-dire une serrure à clé ou analogue, permettant de protéger les différents éléments et systèmes qu'elle renferme de la poussière ou de manipulations inopinées ou par des personnes non autorisées.

Par ailleurs, une découpe dans l'une 61 des portes permet à l'utilisateur d'avoir accès à différents afficheurs, touches de commande ou analogues situés sur un boîtier 63 relié au système de pilotage 5. En particulier, on peut utiliser à cette fin un boîtier 63 de type VIGI 3077 commercialisé par la société Air Liquide Medical Systems qui reçoit toutes les informations des automates du système de pilotage 5 et qui affiche les alarmes destinées aux utilisateurs. Ainsi, l'information du personnel médical, par exemple des informations générales de fonctionnement, peuvent être affichées et visibles depuis l'extérieur de l'armoire 1 grâce à l'utilisation d'un boîtier 63 de type VIGI 3077, et dans les salles de soins par l'intermédiaire de boîtiers de report d'alarmes, par exemple de type VIGI 3003 également commercialisés par Air Liquide Médical Systems.

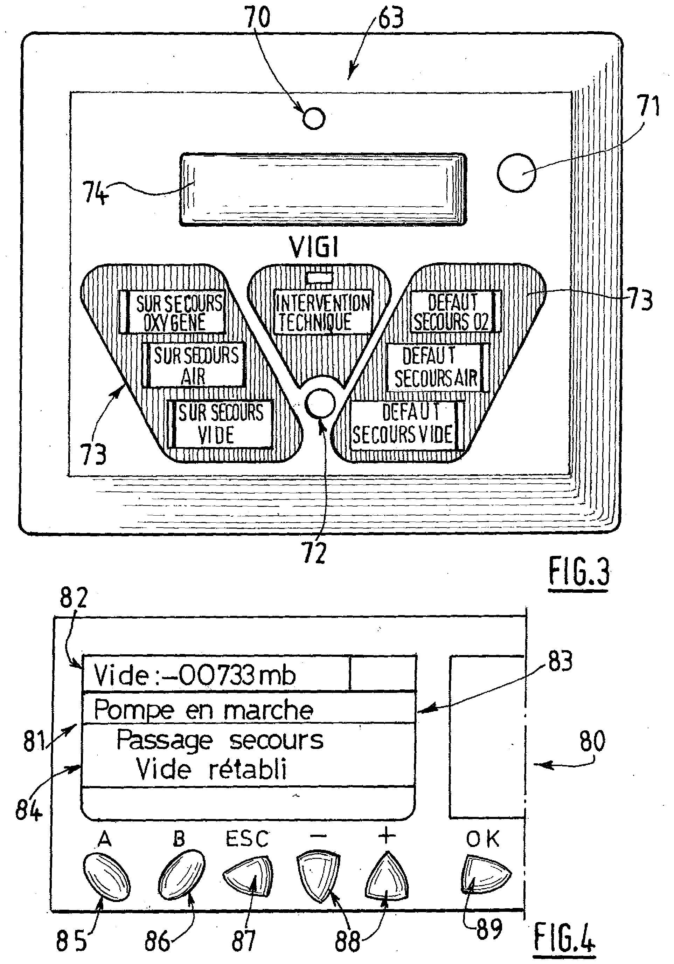

Un boîtier 63 de type VIGI 3077 est illustré en Figure 3. Celui-ci comporte un voyant de bon fonctionnement 70 (voyant vert), un bouton ou touche d'acquittement 71 pour inhiber temporairement une alarme sonore, un bouton ou touche 72 de test pour l'afficheur, les voyants..., des voyants d'alarmes 73 qui clignotent en cas de défaut et un écran d'affichage 74 des différentes alarmes.

Par ailleurs, le système de pilotage 5 peut comprendre un ou plusieurs automates programmables avec calculateur interne et toute sa connectique, notamment des automates de référence commerciale Millenium 3 commercialisés par la société Crouzet.

L'interface homme/machine 80 d'un tel automate est schématisée en Figure 4. Elle comprend un afficheur 81 apte à afficher différentes informations, tel que le niveau de vide ou dépression en aval 82 de l'armoire 1, l'état de fonctionnement 83 de la pompe à vide 4, des messages 84 d'alerte ou d'état de l'installation, ainsi que différentes touches activables par l'utilisateur, notamment une touche (A) de défilement de seuils de pression 85, une touche (B) 86 d'inhibition de pompe à vide 4 (avec touche -) ou de lancement de modification de seuils (avec touche +), une touche 87 d'interruption (ESC) de mode test, des touches 88 de réglage de seuils (+) et (-), et une touche 89 d'acquittement pour la validation des modifications de seuils de dépression.

Une armoire 1 selon l'invention a typiquement une hauteur d'environ 1,5 m à 1,90 m, une largeur d'environ 1,20 m à 1,60 m et une profondeur d'environ 0,40 à 0,70 m. Par exemple, l'armoire représentée sur la Figure 2 à une hauteur de 1,85 m, une largeur de 1,55 m et une profondeur de 0,44 m.

La coque externe formant l'armoire 1 est préférentiellement faite en une tôle d'acier couverte de peinture et comporte une ou plusieurs ouvertures 64, préférentiellement munies de grilles ou analogues, servant à l'aération de l'intérieur de l'armoire 1, comme illustré en Figure 4.

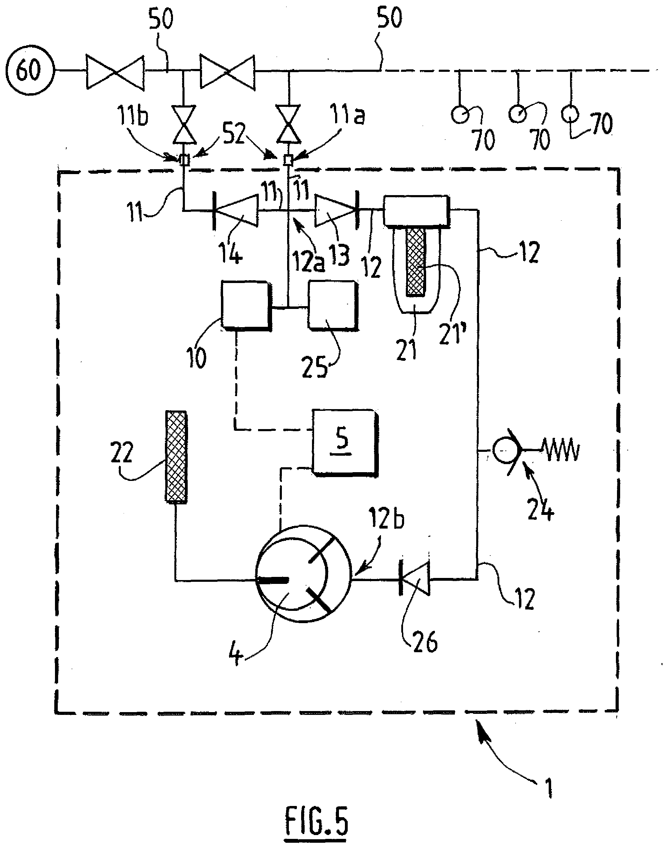

L'armoire 1 permet d'assurer un secours en vide automatiquement et directement sur le réseau secondaire, c'est-à-dire sur la partie de réseau 50 située entre l'armoire 1 et les sites utilisateurs 70, comme illustré en Figure 5. Ceci permet d'assurer le maintien d'une dépression à un niveau adéquat, i.e. à une pression inférieure à la pression atmosphérique, uniquement dans la partie du réseau concernée par la défaillance.

Dans tous les cas, comme illustré en Figure 5, l'installation de mise sous vide de l'invention comporte, agencés dans l'armoire 1, une ligne principale de vide 11 comprenant un orifice d'entrée 11a et un orifice de sortie 11b de vide. De préférence, ces orifices 11a, 11b sont situés au niveau des extrémités de la ligne principale 11 qui comprennent des raccords 52 permettant la connexion de ladite ligne principale 11 à la canalisation de vide 50 du réseau de vide parcourant le bâtiment hospitalier, par exemple des raccords classiques vissés. De cette manière, l'armoire 1 peut aisément être raccordée en tout point du réseau de vide moyennant une simple dérivation de la canalisation 50.

Pour assurer un maintien du niveau de vide souhaité, en cas de défaillance du réseau 50, notamment de tout ou partie des pompes à vide 60, une ligne annexe 12 de soutirage de vide, telle une canalisation souple, est raccordée fluidiquement, via son extrémité amont 12a, à la ligne principale de vide 11 et , via son extrémité aval 12b, à la pompe à vide 5 agencée dans l'armoire 1 de manière à pouvoir aspirer l'air présent dans la ligne principale 11 à l'aide de la pompe à vide 5 et à ainsi mettre et maintenir cette ligne 11 en dépression (< 1 bar).

La ligne principale 11 comporte, quant à elle, un premier dispositif anti-retour 14 permettant l'aspiration de l'air dans le sens allant de l'armoire 1 vers la ou les pompes à vide 60 agencée sur le réseau 50 mais s'opposant à une aspiration dans le sens opposé. La ligne annexe 12 de soutirage de vide comprend également un dispositif anti-retour 13 agencé dans le sens opposé du dispositif anti-retour 14, c'est-à-dire permettant l'aspiration de l'air dans le sens contraire, à savoir notamment dans la ligne 12, en direction de la pompe à vide 4 de l'armoire 1. La présence de ces clapets anti-retour est primordiale pour éviter que les aspirations ou circulations de « vide » ne se fassent dans des portions non souhaitées des canalisations ou analogues.

Le passage des lignes de vide dans l'armoire 1 constitue un point bas du réseau de vide au niveau duquel sont susceptibles de s'accumuler des liquides aspirés. De là, il est prévu sur la ligne annexe 12 de soutirage de vide, un récipient de récupération 21 de liquide, encore appelé pot de vide, servant à recueillir lesdits liquides et permettant leur élimination facile. Ceci constitue un avantage d'une installation selon l'invention.

Dans le cas présent, le récipient de récupération 21 de liquide est équipé d'un filtre 21', en particulier un filtre bactérien permettant d'éliminer les microorganismes susceptibles d'avoir été entraînés par l'aspiration de la ou des pompes à vide. Toutefois, le filtre 21' pourrait être aussi situé hors et être indépendant du récipient 21. On peut éventuellement prévoir qu'une alarme avertisse l'utilisateur lorsque le récipient 21 est plein de liquide et doit être vidé ; toutefois, ceci n'est qu'optionnel.

Par ailleurs, un autre dispositif anti-retour 26 est agencé sur la ligne annexe 12 de soutirage de vide, à proximité de la pompe 4 à vide, de manière à pallier toute remontée de pression dans la ligne 12 et vers les canalisations 50, en cas de défaillance de la pompe 4 notamment.

En mode de fonctionnement normal, la gestion du secours automatique de vide est pilotée par un capteur de pression analogique 10 dont la prise de pression est reliée à la ligne principale 11 et qui est lui-même relié électriquement au système de pilotage 5 de manière à lui transmettre des signaux de mesure de vide, c'est-à-dire de dépression, régnant dans la ligne principale 11 et donc dans la canalisation 50.

Lorsque la dépression régnant dans la ligne principale 11 est à un niveau de pression suffisamment bas, c'est-à-dire à une valeur instantanée de vide inférieure à par exemple -340 mbar (i.e. le signe « - » signifie qu'il s'agit d'une pression de 0,340 bar en dessous de la pression atmosphérique, c'est-à-dire une dépression de 340 mbar), le capteur de pression 10 transmet cette mesure au système de pilotage 5 qui la compare avec une valeur seuil préfixée (par exemple -340 mbar) et en conclut un fonctionnement correct de l'installation. Dans ce cas, le système de pilotage 5 ne déclenche aucune alarme. Le clapet anti-retour 13 de la ligne 12 annexe de soutirage de vide est alors en position fermée, isolant ainsi la pompe de secours 4 du reste du réseau 50. Les mesures de pression instantanée opérées par le capteur 10 peuvent être faites en continu ou alors de manière discontinue, à des intervalles de temps donnés.

Lorsque le vide vient à manquer, c'est-à-dire que le niveau de pression remonte progressivement dans le réseau 50, cette remontée de pression est mesurée par le capteur de pression 10 qui transmet un signal de mesure de pression instantanée au système de pilotage 5 qui la compare avec la valeur seuil préfixée (par exemple -340 mbar) et conclut à un dysfonctionnement de l'installation lorsque cette pression instantanée atteint par exemple cette valeur seuil de -340 mbar. Une alarme sonore et/ou visuelle est alors déclenchée pour alerter l'utilisateur et s'affiche sur le boitier 63 de la Figure 4.

Ensuite, lorsque le niveau de pression instantanée remonte encore et atteint la valeur critique de -300 mbar, le capteur 10 de pression transmet cette valeur mesurée au système de pilotage 5 qui commande alors la pompe à vide 4 de secours pour une durée donnée, en général plusieurs minutes, par exemple 5 minutes. Le réglage de cette temporisation se fait au niveau du système de pilotage 5.

La mise en route de la pompe 4 engendre alors un soutirage de gaz dans la ligne 12 annexe puis dans la partie du réseau 50 de vide située entre l'armoire 1 et les sites utilisateurs 70, ce qui rétablit alors la pression du réseau à une valeur adéquate préfixée, par exemple -700 mbar, qui est typiquement la valeur limite imposée par la soupape dite « casse vide » 24 de la pompe 4. Dans ce cas, le clapet anti-retour 14 est fermé en isolant ainsi le réseau 50 secouru du reste du réseau de vide du bâtiment, alors que le clapet anti-retour 13 de la ligne annexe 12 est ouvert, ainsi que le clapet anti-retour 26.

L'air aspiré par la pompe 4 passe par le filtre bactérien 21' de type F65 équipant le récipient de récupération de liquide 21, avant d'être refoulé à l'atmosphère ambiante, via un autre filtre 22 bactérien situé en sortie de pompe 4.

Pendant tout le soutirage d'air par la pompe 4, l'utilisateur est informé en permanence par le système de pilotage 5 et le boîtier 63, comme détaillé ci-après.

Lorsque la pompe à vide 4 est commandée pour s'arrêter après une durée préfixée de fonctionnement, par exemple après 5 minutes de fonctionnement, si le dysfonctionnement ayant provoqué la remontée de pression a été éliminé, alors l'armoire 1 de l'invention repasse en mode veille. Sinon, la pompe à vide 4 est redémarrée par le système de pilotage 5 pour une autre durée donnée, par exemple 5 minutes additionnelles.

En d'autres termes, lorsqu'on détermine que la valeur de dépression instantanée mesurée est supérieure à la valeur-seuil de dépression préfixée, par exemple -340 mbar, la mise en route et le fonctionnement de la pompe à vide de l'installation n'est pas forcément commandée immédiatement mais seulement lorsque le capteur mesure une valeur de dépression instantanée supérieure ou égale à une valeur critique préfixée, par exemple -200 mbar, comprise entre ladite valeur-seuil de dépression (i.e. -340 mbar par exemple) et la pression atmosphérique. Bien entendu, il serait également possible de mettre en route la pompe 4 dès dépassement de la valeur-seuil de dépression, à savoir ici -340 mbar, sans attendre une élévation de pression jusqu'à la valeur critique (i.e. -200 mbar ici).

Les valeurs de pressions sont mémorisées et/ou enregistrées au sein du système de pilotage 5.

Par ailleurs, une alarme spécifique du boîtier 63 prévient l'utilisateur en cas de problème de la pompe à vide 4 de secours, notamment si celle-ci disjoncte.

Le système de pilotage 5 et le capteur 10 notamment sont alimentés en courant électrique par le secteur, c'est-à-dire fonctionnement sur 220 V et 16 A. Toutefois, faire fonctionner l'installation à un autre voltage et/ou ampérage est tout à fait possible moyennant quelques adaptations de routine, notamment via l'utilisation de transformateurs ou convertisseurs de courant.

Le système de pilotage 5 commande donc le démarrage de la pompe à vide 4 et le déclenchement des alarmes en réponse aux mesures de pression opérée par le capteur 10. En cas de détection d'un niveau de dépression inapproprié, une alarme sonore et/ou visuelle peut être déclenchée pour avertir le personnel soignant du problème rencontré.

L'armoire 1 comporte également un vaccuostat de secours 25 qui permet, en cas de dysfonctionnement du capteur 10 notamment conduisant à une non détection de la remontée de pression à -300 mbar, de détecter automatiquement toute remontée de pression intempestive à une valeur seuil donnée, par exemple à -270 mbar, et de commander alors directement la pompe à vide 4 pendant une durée préfixée, par exemple pendant 5 minutes, de manière à faire baisser la pression et ce, indépendamment du système de pilotage 5. Ce cycle est recommencé autant de fois que nécessaire tant que le dysfonctionnement du capteur persiste et que la pression remonte intempestivement dans le circuit.

Dit autrement, le dispositif vaccuostat 25 est apte à et conçu pour commander automatiquement, un démarrage et un fonctionnement de la pompe à vide 4, pendant une durée déterminée, en cas de remontée de pression au sein de la ligne principale 11 au-delà d'une valeur seuil préfixée. Ce vaccuostat 25 comporte une prise de pression raccordée à la ligne 11 principale.

Plus généralement, l'armoire 1 de l'invention permet de réaliser automatiquement un secours efficace et rapide, pendant quelques heures, d'un réseau de distribution de vide au sein d'un bâtiment hospitalier et permettre ainsi, par exemple qu'une opération chirurgicale ou similaire puisse se terminer normalement.

## Revendications

1. Installation de distribution de vide comprenant :
- une ligne principale de vide (11) comprenant un orifice d'entrée (11a) et un orifice de sortie (11b),
- au moins une pompe à vide (4),
- une ligne annexe de soutirage de vide (12) raccordée fluidiquement, via une extrémité amont (12a), à la ligne principale de vide (11) et via une extrémité aval (12b), à la pompe à vide (4),
- un capteur de pression (10) comportant une prise de pression gazeuse raccordée fluidiquement à la ligne principale de vide (11), et
- un système de pilotage (5) raccordé au capteur de pression (10) et commandant ladite une pompe à vide (4) en réponse à au moins une mesure de pression opérée par le capteur de pression (10) au sein de la ligne principale de vide (11),
dans laquelle au moins une partie de la ligne principale de vide (11), la ligne annexe de soutirage (12) de vide, la pompe à vide (4), le capteur de pression (10), et le système de pilotage (5) sont compris dans une armoire (1),
dans laquelle le système de pilotage (5) comporte au moins un automate programmable, et dans laquelle un boîtier (63), relié au système de pilotage (5), reçoit toutes les informations du ou des automates du système de pilotage (5),
l'installation étant **caractérisée en ce que**
- le système de pilotage (5) est conçu pour et apte à :
i) démarrer la pompe à vide (4) lorsque la pression mesurée par le capteur (10) de pression est supérieure à une valeur-seuil de pression préfixée, ladite valeur seuil de pression étant inférieure à 1 bar, et
ii) arrêter la pompe à vide (4) après une durée de fonctionnement préfixée,
- et un vaccuostat de secours (25) permet, en cas de dysfonctionnement du capteur de pression (10), de détecter automatiquement toute remontée de pression à une valeur-seuil préfixée et de commander la pompe à vide (4) pendant une durée de fonctionnement préfixée, indépendamment du système de pilotage (5).

2. Installation selon la revendication précédente, **caractérisée en ce que** le système de pilotage est conçu pour et apte à démarrer la pompe à vide lorsque la pression mesurée par le capteur excède une valeur-seuil préfixée et une valeur de pression critique préfixée comprise entre la valeur-seuil de dépression et la pression atmosphérique.

3. Installation selon au moins l'une des revendications précédentes, **caractérisée en ce que** la ligne annexe de soutirage de vide (12) comprend au moins un dispositif de filtration (21 ') de gaz, en particulier un filtre anti-bactérien.

4. Installation selon au moins l'une des revendications précédentes, **caractérisée en ce qu'**elle comporte en outre un récipient de récupération (21) de liquide.

5. Installation selon au moins l'une des revendications précédentes, **caractérisée en ce qu'**une découpe dans l'une des portes (60, 61) de l'armoire (1) permet à l'utilisateur d'avoir accès à différents afficheurs, touches de commande ou analogues situés sur le boîtier (63) relié au système de pilotage (5).

6. Installation selon au moins l'une des revendications précédentes, **caractérisée en ce qu'**elle comprend un premier dispositif anti-retour de gaz (14) agencé sur la ligne principale (11) de vide et/ou un deuxième dispositif anti-retour de gaz (13) agencé sur la ligne annexe de soutirage de vide (12), de préférence au moins un dispositif anti-retours (13, 14) comprend un clapet anti-retour.

7. Installation selon au moins l'une des revendications précédentes, **caractérisée en ce que** le système de pilotage (5) est conçu pour et apte à arrêter la pompe à vide (4) après une durée de fonctionnement préfixée comprise entre 1 minute et 30 minutes.

8. Installation selon au moins l'une des revendications précédentes, **caractérisée en ce que** le vaccuostat comporte une prise de pression raccordée à la ligne principale (11).

9. Installation selon au moins l'une des revendications précédentes, **caractérisée en ce qu'**elle comporte une alarme sonore ou visuelle se déclenchant en réponse à une mesure par le capteur (10) d'une valeur de pression supérieure ou égale à une valeur de pression seuil au sein de la ligne principale de vide (11).

10. Installation selon au moins l'une des revendications précédentes, **caractérisée en ce que** le boîtier (63) reçoit toutes les informations du ou des automates du système de pilotage (5) et affiche les alarmes destinées aux utilisateurs.

11. Canalisation (50) de vide reliant une source de vide (60), en particulier une ou plusieurs pompes à vide (60), à au moins un site utilisateur (70), ladite source de vide étant raccordée fluidiquement à une entrée de ladite canalisation (50) de manière à pouvoir mettre ladite canalisation (50) en dépression, **caractérisée en ce que** ladite canalisation (50) comprend une installation selon l'une des revendications précédentes, et **en ce que** ladite canalisation est raccordée au moins à une entrée (11a) et à au moins une sortie (11b) d'une ligne principale (11) de vide de cette installation.

12. Procédé de pilotage d'une installation selon l'une des revendications 1 à 10 agencée sur une canalisation (50) de vide selon la revendication 11, **caractérisé en ce qu'**il comporte les étapes de :
a) déterminer au moyen d'au moins un capteur (10) de pression, la dépression instantanée régnant dans la ligne principale (11) de vide de l'installation de distribution de vide,
b) comparer au moyen du système de pilotage (5), la valeur de dépression instantanée déterminée à l'étape a) avec au moins une valeur-seuil de vide préfixée, ladite valeur-seuil de vide préfixée étant inférieure à la pression atmosphérique, et
c) commander au moyen du système de pilotage (5), une mise en route et un fonctionnement de la pompe à vide (4) de l'installation, lorsqu'à l'étape b), on détermine que la valeur de dépression instantanée mesurée est supérieure à la valeur-seuil de vide préfixée, et extraire du gaz, au moyen de ladite pompe à vide (4), d'au moins une partie de la ligne principale (11) et de la canalisation (50) reliée fluidiquement à ladite ligne principale (11),
d) stopper la pompe à vide (4) après une durée donnée,
**caractérisé en ce qu'**en cas de dysfonctionnement du capteur de pression (10), un vaccuostat de secours (25) détecte automatiquement toute remontée de pression à une valeur-seuil préfixée et commande la pompe à vide (4) pendant une durée de fonctionnement préfixée, indépendamment du système de pilotage (5).

13. Procédé selon la revendication 12, **caractérisé en ce que**, lorsqu'à l'étape b), on détermine que la valeur de dépression instantanée mesurée est supérieure à la valeur-seuil de dépression préfixée, on déclenche au moins une alarme sonore ou visuelle, ou les deux.

14. Procédé selon l'une des revendications 12 ou 13, **caractérisé en ce qu'**à l'étape d), la durée donnée est comprise entre 1 et 30 min.

15. Procédé selon l'une des revendications 12 à 14, **caractérisé en ce que**, lorsqu'à l'étape b), on détermine que la valeur de dépression instantanée mesurée est supérieure à la valeur-seuil de dépression préfixée, la mise en route et le fonctionnement de la pompe à vide (4) de l'installation est commandée seulement lorsque le capteur (10) détermine une valeur de dépression instantanée est supérieure ou égale à une valeur critique préfixée, ladite valeur critique préfixée étant comprise entre ladite valeur-seuil de dépression et la pression atmosphérique, et de préférence on répète cycliquement les étapes c) et d) tant que la valeur de dépression mesurée par le capteur (10) est supérieure à la valeur-seuil de vide préfixée.

## Patentansprüche

1. Installation zur Vakuumverteilung, umfassend:
- eine Vakuum-Hauptleitung (11), eine Eingangsöffnung (11a) und eine Ausgangsöffnung (11b) umfassend,
- mindestens eine Vakuumpumpe (4),
- eine Nebenleitung zur Entnahme von Vakuum (12), die fluidtechnisch über ein stromaufwärtiges Ende (12a) an die Vakuum-Hauptleitung (11), und über ein stromabwärtiges Ende (12b) an die Vakuumpumpe (4) angeschlossen ist,
- einen Drucksensor (10), einen Gasdruckanschluss umfassend, der fluidtechnisch an die Vakuum-Hauptleitung (11) angeschlossen ist, und
- ein Steuersystem (5), das an den Drucksensor (10) angeschlossen ist, und die Vakuumpumpe (4) als Reaktion auf mindestens eine Druckmessung steuert, die durch den Drucksensor (10) in der Vakuum-Hauptleitung (11) durchgeführt wird,
wobei mindestens ein Abschnitt der Vakuum-Hauptleitung (11), die Nebenleitung zur Entnahme (12) von Vakuum, die Vakuumpumpe (4), der Drucksensor (10) und das Steuersystem (5) in einem Schrank (1) enthalten sind,
wobei das Steuersystem (5) mindestens einen programmierbaren Automaten umfasst,
und wobei ein Gehäuse (63), das mit dem Steuersystem (5) verbunden ist, sämtliche Informationen des oder der Automaten des Steuersystems (5) empfängt,
wobei die Installation **dadurch gekennzeichnet ist, dass**
- das Steuersystem (5) gestaltet und imstande ist, um:
i) die Vakuumpumpe (4) zu starten, wenn der durch den Drucksensor (10) gemessene Druck größer ist, als ein festgesetzter Druckgrenzwert, wobei der Druckgrenzwert kleiner als 1 bar ist, und
ii) die Vakuumpumpe (4) nach einer festgesetzten Betriebsdauer anzuhalten,
- und es ein Sicherheits-Vakuumschalter (25) im Falle einer Funktionsstörung des Drucksensors (10) ermöglicht, automatisch jeden Druckanstieg auf einen festgesetzten Grenzwert zu detektieren, und die Vakuumpumpe (4) während einer festgesetzten Betriebsdauer unabhängig von dem Steuersystem (5) zu steuern.

2. Installation nach dem vorstehenden Anspruch, **dadurch gekennzeichnet, dass** das Steuersystem gestaltet und imstande ist, um die Vakuumpumpe zu starten, wenn der durch den Sensor gemessene Druck einen festgesetzten Grenzwert und einen festgesetzten kritischen Druckwert übersteigt, der zwischen dem Unterdruck-Grenzwert und dem atmosphärischen Druck enthalten ist.

3. Installation nach mindestens einem der vorstehenden Ansprüche, **dadurch gekennzeichnet, dass** die Nebenleitung zur Entnahme von Vakuum (12) mindestens eine Gasfiltervorrichtung (21'), im Speziellen einen Bakterienfilter umfasst.

4. Installation nach mindestens einem der vorstehenden Ansprüche, **dadurch gekennzeichnet, dass** sie weiter einen Auffangbehälter (21) von Flüssigkeit umfasst.

5. Installation nach mindestens einem der vorstehenden Ansprüche, **dadurch gekennzeichnet, dass** es ein Ausschnitt in einer der Türen (60, 61) des Schranks (1) dem Benutzer ermöglicht, Zugriff auf verschiedene Anzeigegeräte, Steuertasten oder ähnliches zu haben, die sich auf dem Gehäuse (63) befinden, das mit dem Steuersystem (5) verbunden ist.

6. Installation nach mindestens einem der vorstehenden Ansprüche, **dadurch gekennzeichnet, dass** sie eine erste Rückschlagvorrichtung für Gas (14) umfasst, die in der Vakuum-Hauptleitung (11) angeordnet ist, und/ oder eine zweite Rückschlagvorrichtung für Gas (13), die in der Nebenleitung zur Entnahme von Vakuum (12) angeordnet ist, wobei vorzugsweise mindestens eine Rückschlagvorrichtung für Gas (13, 14) ein Rückschlagventil umfasst.

7. Installation nach mindestens einem der vorstehenden Ansprüche, **dadurch gekennzeichnet, dass** das Steuersystem (5) gestaltet und imstande ist, um die Vakuumpumpe (4) nach einer festgesetzten Betriebsdauer zwischen 1 Minute und 30 Minuten anzuhalten.

8. Installation nach mindestens einem der vorstehenden Ansprüche, **dadurch gekennzeichnet, dass** der Vakuumschalter einen Druckanschluss umfasst, der an die Hauptleitung (11) angeschlossen ist.

9. Installation nach mindestens einem der vorstehenden Ansprüche, **dadurch gekennzeichnet, dass** sie einen akustischen oder optischen Alarm umfasst, der als Reaktion auf eine Messung durch den Sensor (10) mit einem Druckwert größer oder gleich einem Druckgrenzwert in der Vakuum-Hauptleitung (11) auslöst.

10. Installation nach mindestens einem der vorstehenden Ansprüche, **dadurch gekennzeichnet, dass** das Gehäuse (63) sämtliche Informationen des oder der Automaten des Steuersystems (5) empfängt und die für die Benutzer bestimmten Alarme anzeigt.

11. Vakuum-Rohrleitung (50), die eine Vakuumquelle (60), im Speziellen eine oder mehrere Vakuumpumpen (60), mit mindestens einem Benutzerstandort (70) verbindet, wobei die Vakuumquelle fluidtechnisch an einen Eingang der Vakuum-Rohrleitung (50) angeschlossen wird, um die Rohrleitung (50) unter Unterdruck setzen zu können, **dadurch gekennzeichnet, dass** die Rohrleitung (50) eine Installation nach einem der vorstehenden Ansprüche umfasst, und dadurch, dass die Rohrleitung mindestens an einen Eingang (11a) und mindestens einen Ausgang (11b) einer Vakuum-Hauptleitung (11) dieser Installation angeschlossen ist.

12. Verfahren zum Steuern einer Installation nach einem der Ansprüche 1 bis 10, die in einer Vakuum-Rohrleitung (50) nach Anspruch 11 angeordnet ist, **dadurch gekennzeichnet, dass** es die Schritte umfasst:
a) Bestimmen anhand von mindestens einem Drucksensor (10) des momentanen Unterdrucks, der in der Vakuum-Hauptleitung (11) der Installation zur Vakuumverteilung vorherrscht,
b) Vergleichen, anhand des Steuersystems (5), des momentanen Unterdruckwerts, der in Schritt a) bestimmt wird, mit mindestens einem festgesetzten Vakuum-Grenzwert, wobei der festgesetzte Vakuum-Grenzwert kleiner als der atmosphärische Druck ist, und
c) Steuern, anhand des Steuersystems (5), einer Inbetriebnahme und eines Betriebs der Vakuumpumpe (4) der Installation, wenn man im Schritt b) bestimmt, dass der gemessene momentane Unterdruckwert größer als der festgesetzte Vakuumgrenzwert ist, und Gas anhand der Vakuumpumpe (4) aus mindestens einem Abschnitt der Hauptleitung (11) und der Rohrleitung (50) zu entziehen, die fluidtechnisch mit der Hauptleitung (11) verbunden ist,
d) die Vakuumpumpe (4) nach einer gegebenen Dauer anzuhalten,
**dadurch gekennzeichnet, dass** im Falle einer Funktionsstörung des Drucksensors (10) ein Sicherheits-Vakuumschalter (25) automatisch jeden Druckanstieg auf einen festgesetzten Grenzwert detektiert, und die Vakuumpumpe (4) während einer festgesetzten Betriebsdauer unabhängig von dem Steuersystem (5) steuert.

13. Verfahren nach Anspruch 12, **dadurch gekennzeichnet, dass** wenn man im Schritt b) bestimmt, dass der gemessene momentane Unterdruckwert größer ist, als der festgesetzte Unterdruck-Grenzwert, man mindestens einen akustischen oder optischen Alarm, oder beide, auslöst.

14. Verfahren nach einem der Ansprüche 12 oder 13, **dadurch gekennzeichnet, dass** die gegebene Dauer im Schritt d) zwischen 1 und 30 Min. liegt.

15. Verfahren nach einem der Ansprüche 12 bis 14, **dadurch gekennzeichnet, dass**, wenn man im Schritt b) bestimmt, dass der gemessene momentane Unterdruck-Grenzwert größer als der festgesetzte Unterdruck-Grenzwert ist, die Inbetriebnahme und der Betrieb der Vakuumpumpe (4) nur gesteuert wird, wenn der Sensor (10) einen momentanen Unterdruckwert bestimmt, der größer oder gleich einem festgesetzten kritischen Wert ist, wobei der festgesetzte kritische Wert zwischen dem Unterdruck-Grenzwert und dem atmosphärischen Druck enthalten ist, und man vorzugsweise zyklisch die Schritte c) und d) wiederholt, solange der vom Sensor (10) gemessene Unterdruckwert größer als der festgesetzte Vakuum-Grenzwert ist.

## Claims

1. A vacuum distribution installation comprising:
- a main vacuum line (11) comprising an inlet orifice (11a) and an outlet orifice (11b),
- at least one vacuum pump (4),
- an associated vacuum bleed line (12), fluidly connected via an upstream extremity (12a) to the main vacuum line (11) and via a downstream extremity (12b) to the vacuum pump (4),
- a pressure sensor (10) including a gas pressure tap, fluidly connected to the main vacuum line (11), and
- a control system (5) connected to the pressure sensor (10) and controlling the said one vacuum pump (4) in response to at least one pressure measurement performed by the pressure sensor (10) in the main vacuum line (11),
wherein at least one part of the main vacuum line (11), the associated vacuum bleed line (12), the vacuum pump (4), the pressure sensor (10) and the control system (5) are comprised in a cabinet (1),
wherein the control system (5) includes at least one programmable logic controller,
and wherein
a control box (63), connected to the control system (5) receives the entirety of the data from the one or more control system (5) logic controllers,
the installation being **characterised in that**
- the control system (5) is designed to and is able to:
i) start up the vacuum pump (4) when the pressure measured by the pressure sensor (10) exceeds a pre-set threshold pressure value, said threshold pressure value being lower than 1 bar, and
ii) halt the vacuum pump (4) after a pre-set period of operation,
and a safety vacuum switch (25) enabling, in the event of a malfunction of the pressure sensor (10) the automatic detection of any increase in pressure up to a pre-set threshold value and the controlling of the vacuum pump (4) during a pre-set period of operation, independently of the control system (5).

2. An installation as claimed in the preceding claim, **characterised in that** the control system is designed to and is able to start up the vacuum pump when the pressure measured by the sensor exceeds a pre-set threshold pressure value and a pre-set critical pressure value comprised between the negative-pressure threshold value and the atmospheric pressure.

3. An installation as claimed in any one of the preceding claims, **characterised in that** the associated vacuum bleed line (12) comprises at least one gas filtration device (21'), specifically an antibacterial filter.

4. An installation as claimed in any one of the preceding claims, **characterised in that** it further comprises a liquid recovery container (21).

5. An installation as claimed in any one of the preceding claims, **characterised in that** a cut-out in one of the doors (60, 61) of the cabinet (1) allows the user to access various displays, touch keys or analogue keys located on the control box (63) connected to the control system (5).

6. An installation as claimed in any one of the preceding claims, **characterised in that** it comprises a first gas non-return device (14) disposed on the main vacuum line (11) and/or a second gas non-return device (13) disposed on the associated vacuum bleed line (12), preferably, at least one non-return device (13, 14) comprises a non-return valve.

7. An installation as claimed in any one of the preceding claims, **characterised in that** the control system (5) is designed to and is able to halt the vacuum pump (4) after a pre-set period of operation of between 1 minute and 30 minutes.

8. An installation as claimed in any one of the preceding claims, **characterised in that** the vacuum switch includes a pressure tap connected to the main line (11).

9. An installation as claimed in any one of the preceding claims, **characterised in that** it includes an audible or visible alarm which is triggered in response to a reading by the sensor (10) of a pressure value above or equal to a threshold pressure value within the main vacuum line (11).

10. An installation as claimed in any one of the preceding claims, **characterised in that** the control box (63) receives the entirety of the data from the one or more control system (5) logic controllers and displays the alarms intended for the users.

11. A vacuum pipe (50) linking a vacuum source (60), specifically one or several vacuum pumps (60), to at least one user site (70); said vacuum source being fluidly connected to an inlet of said pipe (50) in such a way as to enable the creation of negative pressure within said pipe (50), **characterised in that** said pipe (50) comprises an installation as claimed in any of the preceding claims, and **in that** said pipe is connected to at least one inlet (11a) and to at least one outlet (11b) of a main vacuum line (11) of this installation.

12. A method for the control of an installation as claimed in any of claims 1 to 10, disposed on a vacuum pipe (50) as claimed in claim 11, **characterised in that** it includes the steps of:
a) determining by means of the at least one pressure sensor (10) the immediate negative-pressure value present in the main vacuum line (11) of the vacuum distribution installation,
b) comparing by means of the control system (5) the immediate negative-pressure value determined in step a) with at least one pre-set vacuum threshold value, said pre-set vacuum threshold value being lower than the atmospheric pressure, and
c) controlling, by means of the control system (5), a start-up and operation of the vacuum pump (4) of the installation, when at step b) it is determined that the immediate negative-pressure value is greater than the pre-set vacuum threshold value, and extracting gas, by means of the said vacuum pump (4), from at least one part of the main line (11) and from the pipe (50) fluidly connected to the said main line (11),
d) halting the vacuum pump (4) on conclusion of a given period,
**characterised in that** in the event of a malfunction of the pressure sensor (10), a safety vacuum switch (25) automatically detects any increase in pressure over a pre-set threshold value and controls the vacuum pump (4) during a pre-set period of operation, independently of the control system (5).

13. A procedure as claimed in claim 12, **characterised in that**, when at step b) it is determined that the immediate negative-pressure value measured is greater than the pre-set negative-pressure threshold value, at least one audible or visible alarm, or both, is triggered.

14. A procedure as claimed in any of claims 12 or 13, **characterised in that** at step d) the given period is comprised between 1 and 30 minutes.

15. A procedure as claimed in any of claims 12 to 14, **characterised in that** when at step b) the immediate negative-pressure value measured is determined to be greater than the pre-set negative-pressure threshold value, the start-up and operation of the vacuum pump (4) of the installation is only initiated when the sensor (10) determines that the immediate negative-pressure value is greater than or equal to a pre-set critical value, said pre-set critical value being comprised between the said negative-pressure threshold value and the atmospheric pressure, and preferably, steps c) and d) are repeated cyclically as long as the negative-pressure value measured by the sensor (10) is greater than the pre-set vacuum threshold value.
